# EUROPEAN PATENT SPECIFICATION

(11) **EP 4 079 293 B1**
(45) Date of publication and mention of the grant of the patent: **11.02.2026**
(21) Application number: 20902925.5
(22) Date of filing: 18.12.2020
(51) Int. Cl.: A61K 9/08, A61K 47/40, A61K 47/26, A61K 9/00, A61K 31/40, A61P 1/04, A61K 9/19

(54) **LIQUID PHARMACEUTICAL COMPOSITION OF 1-(5-(2,4-DIFLUOROPHENYL)-1-((3-FLUOROPHENYL)SULFONYL)-4-METHOXY-1H-PYRROLE-3-YL)-N-METHYLMETHANEAMINE**
FLÜSSIGE PHARMAZEUTISCHE ZUSAMMENSETZUNG VON 1-(5-(2,4-DIFLUORPHENYL)-1-((3-FLUORPHENYL)SULFONYL)-4-METHOXY-1H-PYRROL-3-YL)-N-METHYLMETHANEAMIN
COMPOSITION PHARMACEUTIQUE LIQUIDE DE 1-(5-(2,4-DIFLUOROPHÉNYL)-1-((3-FLUOROPHÉNYL)SULFONYL)-4-MÉTHOXY-1H-PYRROL-3-YL)-N-MÉTHYLMÉTHANEAMINE

(30) Priority: 18.12.2019 KR 20190169734
(43) Date of publication of application: 26.10.2022
(73) Proprietor: Daewoong Pharmaceutical Co., Ltd., Hwaseong-si, Gyeonggi-do 18623 (KR)
(72) Inventor: JUNG, Yeon Jin, Gunpo-si, Gyeonggi-do 15884 (KR); KIM, Gyoung Won, Yongin-si, Gyeonggi-do 17009 (KR); KIM, Gwan Young, Seoul 07292 (KR); JANG, Hye Jung, Gunpo-si, Gyeonggi-do 15825 (KR)
(74) Representative: Germain Maureau
(86) International application number: PCT/KR2020/018648
(87) International publication number: WO 2021/125874

(56) References cited:
- WO-A2-2016/175555
- KR-A- 20070 060 133
- KR-A- 20160 127 646
- US-A1- 2009 036 406
- US-A1- 2014 248 249
- LOFTSSON THORSTEINN ET AL: "Pharmaceutical applications of cyclodextrins. 1. Drug solubilization and stabilization", JOURNAL OF PHARMACEUTICAL SCIENCES, AMERICAN CHEMICAL SOCIETY AND AMERICAN PHARMACEUTICAL ASSOCIATION, US, vol. 85, no. 10, 1 October 1996 (1996-10-01), pages 1017 - 1025, XP002574924, ISSN: 0022-3549, [retrieved on 20000612], DOI: 10.1021/JS950534B
- LOFTSSON THORSTEINN, BREWSTER M E: "Pharmaceutical applications of cyclodextrins. 1. Drug solubilization and stabilization", JOURNAL OF PHARMACEUTICAL SCIENCES, vol. 85, no. 10, 1 October 1996 (1996-10-01), US, pages 1017 - 1025, XP002574924, ISSN: 0022-3549, DOI: 10.1021/js950534b

## Description

### [TECHNICAL FIELD]

The present invention relates to a liquid pharmaceutical composition of 1-(5-(2,4-difluorophenyl)-1-((3-fluorophenyl)sulfonyl)-4-methoxy-1H-pyrrol-3-yl)-N-methylmethanamine, or a pharmaceutically acceptable salt thereof.

### [BACKGROUND ART]

The present invention relates to a liquid pharmaceutical composition suitable for 1-(5-(2,4-difluorophenyl)-1-((3-fluorophenyl)sulfonyl)-4-methoxy-1H-pyrrol-3-yl)-N-methylmethanamine. The above compound is a compound described in Korean Patent Registration No. 10-1613245, and have excellent anti-ulcer activity (i.e., proton pump inhibitory activity, etc.) and eradication activity against Helicobacter pylori (H. pylori) and thus are useful in the prevention and treatment of gastrointestinal tract ulcer, gastritis, reflux esophagitis or gastrointestinal damage caused by H. pylori.

The above compound is a compound that exhibits very low water solubility, and in order to produce it in a liquid pharmaceutical composition, such as an injectable pharmaceutical composition, an excessive amount of a solubilizer and an organic solvent are needed. However, pharmaceutical compositions containing a large amount of a solubilizer and an organic solvent have a problem that hypersensitivity may occur when administering the active component at a high dose. That is, generally, in a drug having low water solubility, in order to develop a liquid formulation having excellent solubility and stability at the same time, it is necessary to study a combination of various components other than the drug.

Solubilizers commonly used for liquid pharmaceutical compositions include ethanol, polysorbate, and the like, but these compounds may have harmful effects on the human body, whereby it is necessary to develop a liquid pharmaceutical composition having excellent solubility and stability at the same time without using such a solubilizer.

Therefore, the present inventors have intensively studied to overcome the problems caused by the solubilizer and organic solvent that are excessively used for solubilizing a conventional poorly soluble drug as a liquid pharmaceutical composition of 1-(5-(2,4-difluorophenyl)-1-((3-fluorophenyl)sulfonyl)-4-methoxy-1H-pyrrol-3-yl)-N-methylmethanamine, and to improve the stability of the liquid pharmaceutical composition, and as a result, found that the above problems can be solved by using specific stabilizers and bulking agents for freeze-drying as described below, thereby completing the present invention.

### [DETAILED DESCRIPTION OF THE INVENTION]

### [Technical Problem]

It is an object of the present invention to provide a liquid pharmaceutical composition of 1-(5-(2,4-difluorophenyl)-1-((3-fluorophenyl)sulfonyl)-4-methoxy-1H-pyrrol-3-yl)-N-methylmethanamine, or a pharmaceutically acceptable salt thereof.

### [Technical Solution]

In order to achieve the above objects, according to the present invention, there is provided a liquid pharmaceutical composition comprising a compound represented by the following Chemical Formula 1, or a pharmaceutically acceptable salt thereof; and a bulking agent for freeze-drying; cyclodextrin; and a solvent,
wherein
the cyclodextrin is beta-cyclodextrin, or gamma-cyclodextrin,
the cyclodextrin is contained in an amount of 3.0 to 25.0 parts by weight based on 1 part by weight of the compound represented by Chemical Formula 1 or a pharmaceutically acceptable salt thereof,
the bulking agent for freeze-drying is D-mannitol, sucrose, sorbitol, or trehalose,
the bulking agent for freeze-drying is contained in an amount of 3.0 to 25.0 parts by weight based on 1 part by weight of the compound represented by Chemical Formula 1 or a pharmaceutically acceptable salt thereof,
the solvent is distilled water, water for injection, acetate buffer, or physiological saline, and [Chemical Formula 1] is

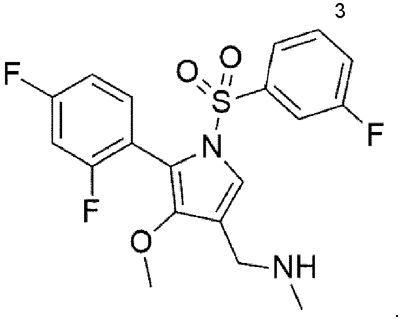

The chemical name of the compound represented by the following Chemical Formula 1 is 1-(5-(2,4-difluorophenyl)-1-((3-fluorophenyl)sulfonyl)-4-methoxy-1H-pyrrol-3-yl)-N-methylmethanamine, which is a compound described in Korean Patent Registration No. 10-1613245.

The above compound is an active component that exhibits pharmacological effects of the liquid pharmaceutical composition according to the present invention, and has excellent anti-ulcer activity (i.e., proton pump inhibitory activity, etc.) and eradication activity against Helicobacter pylori and GPCR inhibitory action, and thus are useful in the prevention and treatment of gastrointestinal tract ulcer, gastritis, reflux esophagitis or gastrointestinal damage caused by H. pylori. Further, the above compound as well as a pharmaceutically acceptable salt of the compound can also be used in the present invention.

Further, in addition to the compound represented by Chemical Formula 1, a pharmaceutically acceptable salt thereof can be used in the present invention. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively non-toxic and harmless to a patient and activates effectively and whose side effects do not degrade the beneficial efficacy of the above compound.

Pharmaceutically acceptable salts can be obtained by conventional methods using inorganic or organic acids. For example, the pharmaceutically acceptable salt can be prepared by dissolving the compound represented by Chemical Formula 1 in a water-miscible organic solvent, e.g., acetone, methanol, ethanol or acetonitrile, followed by adding an organic acid or an inorganic acid, and filtering and drying the precipitated crystals. Alternatively, it may be prepared by subjecting a solvent or an excessive amount of acid from the acid-added reaction mixture to reduced pressure and then drying the residue, or by adding a different organic solvent and then filtering the precipitated salt. At this time, the preferred salts may include salts derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxyrnaleic acid, benzoic acid, hydroxybenzoic acid, phenylacetic acid, cinnamic acid, salicylic acid, methanesulfonic acid, benzenesulfonic acid or toluenesulfonic acid, and the like.

The compound represented by Chemical Formula 1 or a pharmaceutically acceptable salt thereof has low water solubility. Thus, in order to prepare a liquid pharmaceutical composition, such as an injectable pharmaceutical composition, an excessive amount of a solubilizer and an organic solvent is needed. However, an excessive amount of solubilizer and the like may cause hypersensitivity when administered to a patient. Therefore, in the present invention, instead of not using the solubilizer commonly used for liquid pharmaceutical compositions, the above-mentioned components are used, and therefore, a liquid pharmaceutical composition having excellent solubility and stability of the compound represented by Chemical Formula 1 at the same time can be realized.

Cyclodextrin, which is a component used in the liquid pharmaceutical composition according to the present invention, is a cyclic oligosaccharide in which 6 to 12 glucose molecules form alpha-1,4-glycosidic bonds, and is used as a stabilizer in the present invention. The cyclodextrin is beta-cyclodextrin, or gamma-cyclodextrin, preferably, beta-cyclodextrin. More preferably, the beta-cyclodextrin is (2-hydroxypropyl)-beta-cyclodextrin or sulfobutylether-beta-cyclodextrin, and the English abbreviations are 'HP-β-CD' and 'SBE-β-CD', respectively.

Among the stabilizers commonly used in liquid pharmaceutical compositions, the cyclodextrin is suitable for stabilizing the compound represented by Chemical Formula 1 or a pharmaceutically acceptable salt thereof. As in Examples described later, other stabilizers excluding the above are not sufficient to stabilize the compound represented by Chemical Formula 1.

The cyclodextrin is used in an amount of 3.0 to 25.0 parts by weight based on 1 part by weight of the compound represented by Chemical Formula 1 or a pharmaceutically acceptable salt thereof. When the content is less than 3.0 parts by weight, it is not sufficient to stabilize the compound represented by Chemical Formula 1, which causes a problem that rehydration of the liquid pharmaceutical composition is difficult or total related compounds increase during long-term storage. Further, when the content is more than 25.0 parts by weight, the amount of the stabilizer used is excessively high, which may increase the viscosity of the liquid pharmaceutical composition or may cause hypersensitivity when administered to a patient.

More preferably, the content of the cyclodextrin is 3.5 parts by weight or more, 4.0 parts by weight or more, or 4.5 parts by weight or more; and 20.0 parts by weight or less, 19.0 parts by weight or less, 18.0 parts by weight or less, 17.0 parts by weight or less, 16.0 parts by weight or less, 15.0 parts by weight or less, 14.0 parts by weight or less, 13.0 parts by weight or less, 12.0 parts by weight or less, 11.0 parts by weight or less, or 10.0 parts by weight or less, based on 1 part by weight of the compound represented by Chemical Formula 1 or a pharmaceutically acceptable salt thereof.

The liquid pharmaceutical composition according to the present invention further comprises a bulking agent for freeze-drying. Generally, the liquid pharmaceutical composition is mass-produced, then frozen, stored and distributed under reduced pressure, which can enhance the stability of the active ingredient and improve the long-term storage stability. Therefore, the stability of the active compound should be maintained during the freeze-drying process, and thus, in the present invention, a bulking agent for freeze-drying may be additionally included. The bulking agent for freeze-drying is D-mannitol, sucrose, sorbitol, or trehalose, preferably D-mannitol.

The bulking agent for freeze-drying is used in an amount of 3.0 to 25.0 parts by weight based on 1 part by weight of the compound represented by Chemical Formula 1 or a pharmaceutically acceptable salt thereof. When the content is less than 3.0 parts by weight, it is not sufficient to stabilize the compound represented by Chemical Formula 1, which causes a problem that rehydration of the liquid pharmaceutical composition is difficult or related compounds increase during long-term storage. Further, when the content is more than 25.0 parts by weight, the amount of the bulking agent for freeze-drying used is excessively high, which may increase the viscosity of the liquid pharmaceutical composition or may cause hypersensitivity when administered to a patient.

More preferably, the content of the bulking agent for freeze-drying is 3.5 parts by weight or more, 4.0 parts by weight or more, or 4.5 parts by weight or more; and 20.0 parts by weight or less, 15.0 parts by weight or less, 13.0 parts by weight or less, 10.0 parts by weight or less, 9.0 parts by weight or less, 8.0 parts by weight or less, 7.0 parts by weight or less, or 6.0 parts by weight or less, based on 1 part by weight of the compound represented by Chemical Formula 1 or a pharmaceutically acceptable salt thereof.

Preferably, the bulking agent for freeze-drying is used in an amount of 0.5 to 5.0 parts by weight based on 1 part by weight of the cyclodextrin. More preferably, the content of the bulking agent for freeze-drying is 0.6 parts by weight or more, 0.7 parts by weight or more, or 0.8 or more; and 4.5 parts by weight or less, 4.0 parts by weight or less, 3.5 parts by weight or less, 3.0 parts by weight or less, 2.5 parts by weight or less, 2.3 parts by weight or less, 2.0 parts by weight or less, 1.9 parts by weight or less, 1.8 parts by weight or less, 1.7 parts by weight or less, 1.6 parts by weight or less, 1.5 parts by weight or less, 1.4 parts by weight or less, 1.3 parts by weight or less, or 1.2 parts by weight or less, based on 1 part by weight of the cyclodextrin.

Preferably, the liquid pharmaceutical composition according to the present invention is an injectable pharmaceutical composition. Meanwhile, in order to prepare the pharmaceutical composition in liquid form, a conventional solvent in the art to which the present invention pertains can be used. The solvent of the liquid pharmaceutical composition is distilled water, water for injection, acetate buffer, or physiological saline.

Preferably, the compound represented by Chemical Formula 1 or a pharmaceutically acceptable salt thereof is contained at a concentration of 1 to 30 mg/mL in the liquid pharmaceutical composition. That is, the content of the compound represented by Chemical Formula 1 or a pharmaceutically acceptable salt thereof can be defined as a value obtained by dividing the content (mg) of the compound represented by Chemical Formula 1 or a pharmaceutically acceptable salt thereof by the total volume (mL) of the liquid pharmaceutical composition.

More preferably, the compound represented by Chemical Formula 1 or a pharmaceutically acceptable salt thereof is contained at a concentration of 2 mg/mL or more, 3 mg/mL or more, 4 mg/mL or more, or 5 mg/mL or more; and 29 mg/mL or less, 28 mg/mL or less, 27 mg/mL or less, 26 mg/mL or less, 25 mg/mL or less, 24 mg/mL or less, 23 mg/mL or less, 22 mg/mL or less, 21 mg/mL or less, 20 mg/mL or less, 19 mg/mL or less, 18 mg/mL or less, 17 mg/mL or less, 16 mg/mL or less, or 15 mg/mL or less in the liquid pharmaceutical composition.

Preferably, the pH of the liquid pharmaceutical composition according to the present invention is 3.0 to 7.0, more preferably 3.5 to 6.5, still more preferably 4.0 to 6.0. The pH may be adjusted as a pH adjuster, and a conventional acid or alkali in the art to which the present invention belongs can be used as the pH adjuster. As an example of the pH adjuster, any one or more of hydrochloric acid, phosphoric acid, sodium hydroxide, potassium hydroxide, potassium monohydrogen phosphate, potassium dihydrogen phosphate, sodium monohydrogen phosphate, sodium dihydrogen phosphate, sodium carbonate, potassium carbonate, and triethanolamine can be used, without being limited thereto.

Further, if necessary, the liquid pharmaceutical composition according to the present invention may further include a preservative, an antioxidant and the like. The preservative and the antioxidant are not particularly limited as long as they are commonly used in the technical field to which the present invention pertains.

Further, the liquid pharmaceutical composition according to the present invention can be prepared by mixing the above-mentioned components excluding the solvent in a solvent. In this process, the order of addition of each component to a solvent can be adjusted if necessary. Alternatively, all components may be mixed and added to the solvent prior to being added to the solvent.

The prepared liquid pharmaceutical composition may be subjected to sterilization and/or filtration if necessary, and may be freeze-dried for storage and distribution.

### [Advantageous Effects]

The present invention can be usefully used as a liquid pharmaceutical composition of 1-(5-(2,4-difluorophenyl)-1-((3-fluorophenyl)sulfonyl)-4-methoxy-1H-pyrrol-3-yl)-N-methylmethanamine, or a pharmaceutically acceptable salt thereof.

### [DETAILED DESCRIPTION OF THE EMBODIMENTS]

Below, the present invention will be described in more detail to assist in the understanding of the invention. However, the following examples are provided for illustrative purposes only, and should not be construed as limiting the scope of the present invention to these examples. Examples 1-4 are not in the scope of the claims.

### Example 1

As shown in Table 1 below, each preparation liquid adjusted to pH 4.0 was added little by little to 20 mg of the hydrochloride salt of the compound represented by Chemical Formula 1 (hereinafter referred to as 'API'), and the dissolution state was confirmed with the naked eye. Subsequently, the preparation liquid was further added to the experimental group that did not dissolve, and the dissolution state was evaluated. The results are shown in Table 2 below. In Table 2 below, "O" means the case where all are dissolved, and "X" means the case where all are not dissolved and thus a part of API is observed with the naked eye.

**[Table 1]**

| | | **#1-1** | **#1-2** | **#1-3** | **#1-4** | **#1-5** |
|---|---|---|---|---|---|---|
| API | | 20 mg | | | | |
| pH | | 4.0 | | | | |
| Preparation liquid | Water for injection | 1, 2, 4, 5, 10 mL | | | | |
| | Phosphate buffer | | 1, 2, 4, 5, 10 mL | | | |
| | Acetate buffer | | | 1, 2, 4, 5, 10 mL | | |
| | Phthalate buffer | | | | 1, 2, 4, 5, 10 mL | |
| | Citrate buffer | | | | | 1, 2, 4, 5, 10 mL |
| Standard | | | Solubility | | | Visually evaluate the dissolution state at a concentration of 20, 10, 5, 4, 2 mg/mL |

**[Table 2]**

| | | #1-1 | #1-2 | #1-3 | #1-4 | #1-5 |
|---|---|---|---|---|---|---|
| Preparation liquid (pH 4.0) | | Water for injection | Phosphate Bf. | Acetate Bf. | Phthalate Bf. | Citrate Bf. |
| Solubility | 1 mL (20 mg/mL) | X | X | X | X | X |
| | 2 mL (10 mg/mL) | ○ | X | ○ | X | X |
| | 4 mL (5 mg/mL) | ○ | ○ | ○ | X | X |
| | 5 mL (4 mg/mL) | ○ | ○ | ○ | X | X |
| | 10 mL (2 mg/mL) | ○ | ○ | ○ | X | ○ |

As shown in Table 2, it was confirmed that the solubility was excellent in water for injection, phosphate buffer, and acetate buffer as in #1-1 to #1-3.

### Example 2

The following experiments were performed using water for injection in the water for injection, phosphate buffer, and acetate buffer, which had excellent solubility in Example 1.

As shown in Table 3 below, 10 mg of API and each 50 mg of alpha-cyclodextrin (α-CD), gamma-cyclodextrin (γ-CD), (2-hydroxypropyl)-beta-cyclodextrin (HP-β-CD), sulfobutylether-beta-cyclodextrin (SBE-β-CD), Trehalose, PVP K-12, and Poloxamer 188 as stabilizers were dissolved in water for injection (1 mL), to which 0.1N HCl was added to adjust the pH to 4.0.

**[Table 3]**

| | | **#2-1** | **#2-2** | **#2-3** | **#2-4** | **#2-5** | **#2-6** | **#2-7** |
|---|---|---|---|---|---|---|---|---|
| API | | 10 mg | | | | | | |
| Preparation liquid | Water for injection | 1 mL | | | | | | |
| Stabilizer | α-CD | 50 mg | - | - | - | - | - | - |
| | γ-CD | - | 50 mg | - | - | - | - | - |
| | HP-β-CD | - | - | 50 mg | - | - | - | - |
| | SBE-β-CD | - | - | - | 50 mg | - | - | - |
| | Trehalose | - | - | - | - | 50 mg | - | - |
| | PVP K-12 | - | - | - | - | - | 50 mg | - |
| | Poloxamer188 | - | - | - | - | - | - | 50 mg |

Each prepared solution was placed in a 10 mL sample vial and dried by a freeze-drying method. For the freeze-drying, the temperature, time and pressure as shown in Table 4 were sequentially applied.

**[Table 4]**

| | Temperature (°C) | Time (hr) | Pressure (mTorr) |
|---|---|---|---|
| Freezing | -45 | 6 | - |
| Drying | -35 | 2 | 200 |
| | -35 | 12 | 0 |
| | 0 | 12 | 0 |
| | 0 | 6 | 0 |
| | 30 | 3 | 0 |
| | 30 | 6 | 0 |

After the freeze-drying was completed, the freeze-dried sample was stored in a chamber under severe conditions (60°C, 80% RH) for 4 weeks. Each sample was evaluated for rehydration and stability at the initial stage of storage and 4 weeks after storage, respectively, as follows.

### (1) Rehydration

The rehydration solution (water for injection, 0.9% physiological saline, 5% glucose solution, 4 mL each) was administered to the freeze-dried product, which was then observed with the naked eye. It was evaluated as "O" if it was completely dissolved without foreign matters, as "X" if it was not so, and evaluated as "Δ" if it was completely dissolved but then precipitated.

### (2) Stability

The content of related compounds in the freeze-dried product was analyzed by HPLC, and the total amount of total related compounds detected was measured.

The results are shown in Table 5 below.

**[Table 5]**

| | Rehydration | | | | | | Total related compound % | |
|---|---|---|---|---|---|---|---|---|
| | Initial | | | 4-week | | | | |
| | (i) | (ii) | (iii) | (i) | (ii) | (iii) | Initial | 4-week |
| #2-1 | ○ | X | ○ | ○ | X | ○ | 0.15 | 0.23 |
| #2-2 | ○ | ○ | ○ | ○ | ○ | ○ | 0.07 | 0.39 |
| #2-3 | ○ | ○ | ○ | ○ | ○ | ○ | 0.17 | 0.37 |
| #2-4 | ○ | ○ | ○ | ○ | ○ | ○ | 0.09 | 0.22 |
| #2-5 | ○ | X | ○ | X | X | X | 0.10 | 0.40 |
| #2-6 | ○ | X | ○ | ○ | ○ | ○ | 0.10 | 0.57 |
| #2-7 | ○ | X | ○ | X | X | X | 0.10 | 3.19 |
| (i) water for injection, (ii) 0.9% physiological saline, (iii) 5% glucose solution | | | | | | | | |

As shown in Table 5, it was confirmed that the compositions of #2-2 and #2-4 are rehydratable even after being stored under severe conditions for 4 weeks, and exhibit excellent stability with almost no generation of total related compounds.

### Example 3

HP-β-CD having excellent effect as a stabilizer In Example 2 was selected and the following experiment was performed.

As shown in Table 6 below, API and (2-hydroxypropyl)-beta-cyclodextrin (HP-β-CD) were dissolved in water for injection (1 mL), to which 0.1N HCl was added to adjust the pH to 4.0.

**[Table 6]**

| | | #3-1 | #3-2 | #2-3 | #3-3 | #3-4 |
|---|---|---|---|---|---|---|
| API | | 10 mg | | | | |
| Preparation liquid | Water for injection | 1 mL | | | | |
| Stabilizer | HP-β-CD | 25 mg | 35mg | 50mg | I 100mg | 200mg |

Each prepared solution was dried by a freeze-drying method in the same manner as in the previous Example 2, and then the rehydration and stability were evaluated. The results are shown in Table 7 below.

**[Table 7]**

| | Rehydration | | | | | | Total related compounds % | |
|---|---|---|---|---|---|---|---|---|
| | Initial | | | 4-week | | | | |
| | (i) | (ii) | (iii) | (i) | (ii) | (iii) | Initial | 4-week |
| #3-1 | ○ | Δ | ○ | - | - | - | - | - |
| #3-2 | ○ | ○ | ○ | ○ | ○ | ○ | - | - |
| #2-3 | ○ | ○ | ○ | ○ | ○ | ○ | 0.17 | 0.37 |
| #3-3 | ○ | ○ | ○ | ○ | ○ | ○ | 0.16 | 0.35 |
| #3-4 | ○ | ○ | ○ | ○ | ○ | ○ | 0.16 | 0.44 |
| (i) water for injection, (ii) 0.9% physiological saline, (iii) 5% glucose solution | | | | | | | | |

As shown in Table 7, it was confirmed that the compositions #2-3 and #3-2 to #3-4 are rehydratable even after being stored under severe conditions for 4 weeks, and the compositions #2-3 and #3-3 to #3-4 exhibit excellent stability with almost no generation of total related compounds.

### Example 4

HP-β-CD having excellent effect as a stabilizer in Example 2 was selected and the following experiment was performed.

Experiments were conducted with three types of water for injection, acetate buffer, and phosphate buffer derived from Example 1. For this purpose, in addition to the composition of #2-3 of Example 2, a composition was further prepared as shown in Table 8 below, and dissolved in each buffer (1 mL), to which acetic acid and phosphoric acid were added to adjust the pH to 4.0.

**[Table 8]**

| | | #2-3 | #4-1 | #4-2 |
|---|---|---|---|---|
| API | | 10 mg | | |
| Stabilizer | HP-β-CD | 50 mg | | |
| Preparation liquid | Water for injection | 1 mL | - | - |
| | Acetate buffer | - | 1 mL | - |
| | Phosphate buffer | - | - | 1 mL |

Each prepared solution was dried by a freeze-drying method in the same manner as in the previous Example 2, and then the rehydration and stability were evaluated. The results are shown in Table 9 below.

**[Table 9]**

| | Rehydration | | | | | | Total related compounds % | |
|---|---|---|---|---|---|---|---|---|
| | Initial | | | 4-week | | | | |
| | (i) | (ii) | (iii) | (i) | (ii) | (iii) | Initial | 4-week |
| #2-3 | ○ | ○ | ○ | ○ | ○ | ○ | 0.17 | 0.37 |
| #4-1 | ○ | ○ | ○ | ○ | ○ | ○ | 0.06 | 1.27 |
| #4-2 | ○ | △ | ○ | X | X | X | - | - |
| (i) water for injection, (ii) 0.9% physiological saline, (iii) 5% glucose solution | | | | | | | | |

As shown in Table 9, #4-2 had problems with rehydration when stored under severe conditions for 4 weeks. On the other hand, it was confirmed that #2-3 and #4-1 show no abnormality in rehydration when stored under severe conditions for 4 weeks, and exhibit excellent stability with almost no generation of total related compounds.

### Example 5

The properties of the freeze-dried preparation are one of the important factors that can affect the quality in the future, such as rehydration and total related compounds. A solution was prepared with HP-β-CD, which is a stabilizer derived from Examples 1 to 4, and water for injection, to which a bulking agent for freeze-drying was then added and 0.1N HCl was added to adjust the pH to 4.0, thereby preparing a composition.

**[Table 10]**

| | | #5-1 | #5-2 | #5-3 | #5-4 |
|---|---|---|---|---|---|
| API | | 10 mg | | | |
| Preparation liquid | Water for injection | 1 mL | | | |
| Stabilizer | HP-β-CD | 50 mg | | | |
| Bulking agent for freeze-drying | D-mannitol | 50 mg | - | - | - |
| | Sucrose | - | 50 mg | - | - |
| | Sorbitol | - | - | 50 mg | - |
| | Trehalose | - | - | - | 50 mg |

Each prepared solution was dried by a freeze-drying method in the same manner as in the previous Example 2, and then the properties and rehydration were evaluated. The results are shown in Table 11 below.

**[Table 11]**

| | Properties | Rehydration | | |
|---|---|---|---|---|
| | | (i) | (ii) | (iii) |
| #5-1 | White freeze-dried product | ○ | ○ | ○ |
| #5-2 | White freeze-dried product | ○ | ○ | ○ |
| #5-3 | White freeze-dried product | ○ | ○ | ○ |
| #5-4 | White freeze-dried product | ○ | ○ | ○ |
| (i) water for injection, (ii) 0.9% physiological saline, (iii) 5% glucose solution | | | | |

As shown in Table 11, it was confirmed that #5-1 to #5-4 are formulations that maintained the properties of the cake and solubility during rehydration. However, from the viewpoint of the manufacturing process, the optimum drying temperature and time vary depending on the type of bulking agent for freeze-drying. Considering that this is a factor directly related to production efficiency, D-mannitol is most preferable.

### Example 6

In order to confirm the properties, rehydration and stability of the injectable composition according to the content of the bulking agent for freeze-drying D-mannitol derived from Example 5, 0.1N HCl was added to the content shown in Table 12 below to adjust the pH to 4.0, thereby preparing a composition.

**[Table 12]**

| | | #6-1 | #6-2 | #6-3 | #6-4 | #6-5 | #6-6 |
|---|---|---|---|---|---|---|---|
| API | | 10 mg | | | | | |
| Preparation liquid | Water for injection | 1 mL | | | | | |
| Stabilizer | HP-β-CD | 50 mg | | | | | |
| Bulking agent for freeze-drying | D-mannitol | 0 mg | 10mg | 25mg | 50mg | 100mg | 150mg |

Each prepared solution was dried by a freeze-drying method in the same manner as in the previous Example 2, and then the properties, rehydration and stability were evaluated. The results are shown in Table 13 below.

**[Table 13]**

| | Properties | Rehydration | | | | | | Total related compounds % | |
|---|---|---|---|---|---|---|---|---|---|
| | | Initial | | | 4-week | | | | |
| | | (i) | (ii) | (iii) | (i) | (ii) | (iii) | Initial | 4-week |
| #6-1 | Crack | - | - | - | - | - | - | - | - |
| #6-2 | Crack | - | - | - | - | - | - | - | - |
| #6-3 | Crack | - | - | - | - | - | - | - | - |
| #6-4 | Good | ○ | ○ | ○ | ○ | ○ | ○ | 0.12 | 0.18 |
| #6-5 | Good | ○ | ○ | ○ | ○ | ○ | ○ | 0.17 | 0.25 |
| #6-6 | Good | ○ | ○ | ○ | ○ | ○ | ○ | 0.19 | 0.29 |
| (i) water for injection, (ii) 0.9% physiological saline, (iii) 5% glucose solution | | | | | | | | | |

As shown in Table 13, it was confirmed that #6-4 to #6-6 have excellent cake properties without cracks, and are formulations maintaining the solubility during rehydration when stored under severe conditions for 4 weeks. In addition, it was confirmed that they exhibit excellent stability with almost no generation of total related compounds.

### Example 7

The compositions having different pHs in the compositions for injection finally derived from Examples 1 to 6, were prepared as shown in Table 14 below.

**[Table 14]**

| | | #7-1 | #6-4 | #7-2 | #7-3 | #7-4 | #7-5 | #7-6 |
|---|---|---|---|---|---|---|---|---|
| API | | 10 mg | | | | | | |
| Preparation liquid | Water for injection | 1 mL | | | | | | |
| Stabilizer | HP-β-CD | 50 mg | | | | | | |
| Bulking agent for freeze-drying | D-mannitol | 50 mg | | | | | | |
| pH | 0.1N HCl 0.1M NaOH | Proper amount | | | | | | |
| | | 3.0 | 4.0 | 5.0 | 6.0 | 7.0 | 8.0 | 9.0 |

Each prepared solution was dried by a freeze-drying method in the same manner as in the previous Example 2, and then the properties, rehydration and stability were evaluated. The results are shown in Table 15 below.

**[Table 15]**

| | Properties | Rehydration | | | | | | Total related compounds % | |
|---|---|---|---|---|---|---|---|---|---|
| | | Initial | | | 4-week | | | | |
| | | (i) | (ii) | (iii) | (i) | (ii) | (iii) | Initial | 4-week |
| #7-1 | Good | ○ | ○ | O | ○ | ○ | ○ | 0.21 | 0.27 |
| #6-4 | Good | ○ | ○ | O | ○ | ○ | ○ | 0.12 | 0.18 |
| #7-2 | Good | ○ | ○ | O | ○ | ○ | ○ | 0.17 | 0.12 |
| #7-3 | Good | ○ | ○ | O | ○ | ○ | ○ | 0.15 | 0.12 |
| #7-4 | Good | ○ | ○ | O | ○ | ○ | ○ | 0.17 | 0.30 |
| #7-5 | Deposited during manufacture | | | | | | | | |
| #7-6 | Deposited during manufacture | | | | | | | | |
| (i) water for injection, (ii) 0.9% physiological saline, (iii) 5% glucose solution | | | | | | | | | |

As shown in Table 15, it was confirmed that evaluation of # 7-5 and # 7-6 is difficult because API is deposited during manufacture; #6-4 and #7-1 to #7-4 have excellent cake properties without cracks, and are formulations maintaining the solubility during rehydration even after being stored under severe conditions for 4 weeks. In addition, it was confirmed that they exhibit excellent stability with almost no generation of total related compounds.

### Example 8

Compositions having different concentrations in Example 7 were prepared as shown in Table 16 below.

**[Table 16]**

| | | #8-1 | #8-2 | #8-3 | #8-4 | #8-5 | #8-6 | #8-7 |
|---|---|---|---|---|---|---|---|---|
| API | | 20 mg | | | | | | |
| Preparation liquid | Water for injection | 1 mL | | | | | | |
| Stabilizer | HP-β-CD | 100 mg | | | | | | |
| Bulking agent for freeze-drying | D-mannitol | 100 mg | | | | | | |
| pH | 0.1N HCl 0.1M NaOH | Proper amount | | | | | | |
| | | 3.0 | 4.0 | 5.0 | 6.0 | 7.0 | 8.0 | 9.0 |

Each prepared solution was dried by a freeze-drying method in the same manner as in the previous Example 2, and then the properties, rehydration and stability were evaluated. The results are shown in Table 17 below.

**[Table 17]**

| | Properties | Rehydration | | | | | | Total related compounds % | |
|---|---|---|---|---|---|---|---|---|---|
| | | Initial | | | 4-week | | | | |
| | | (i) | (ii) | (iii) | (i) | (ii) | (iii) | Initial | 4-week |
| #8-1 | Good | ○ | ○ | ○ | ○ | ○ | ○ | 0.09 | 0.13 |
| #8-2 | Good | ○ | ○ | ○ | ○ | ○ | ○ | 0.08 | 0.13 |
| #8-3 | Good | ○ | ○ | ○ | ○ | ○ | ○ | 0.09 | 0.15 |
| #8-4 | Good | ○ | ○ | ○ | ○ | ○ | ○ | 0.07 | 0.22 |
| #8-5 | Good | ○ | ○ | ○ | ○ | ○ | ○ | 0.08 | 0.31 |
| #8-6 | Deposited during manufacture | | | | | | | | |
| #8-7 | Deposited during manufacture | | | | | | | | |
| (i) water for injection, (ii) 0.9% physiological saline, (iii) 5% glucose solution | | | | | | | | | |

As shown in Table 17, it was confirmed that evaluation of #8-6 and #8-7 is difficult because API is deposited during manufacture; #8-1 to #8-5 have excellent cake properties without cracks, and are formulations maintaining the solubility during rehydration even after being stored under severe conditions for 4 weeks. In addition, it was confirmed that they exhibit excellent stability with almost no generation of total related compounds.

## Claims

1. A liquid pharmaceutical composition comprising a compound represented by the following Chemical Formula 1, or a pharmaceutically acceptable salt thereof; a bulking agent for freeze-drying; cyclodextrin; and a solvent,
wherein
the cyclodextrin is beta-cyclodextrin, or gamma-cyclodextrin,
the cyclodextrin is contained in an amount of 3.0 to 25.0 parts by weight based on 1 part by weight of the compound represented by Chemical Formula 1 or a pharmaceutically acceptable salt thereof,
the bulking agent for freeze-drying is D-mannitol, sucrose, sorbitol, or trehalose,
the bulking agent for freeze-drying is contained in an amount of 3.0 to 25.0 parts by weight based on 1 part by weight of the compound represented by Chemical Formula 1 or a pharmaceutically acceptable salt thereof,
the solvent is distilled water, water for injection, acetate buffer, or physiological saline, and
[Chemical Formula 1] is

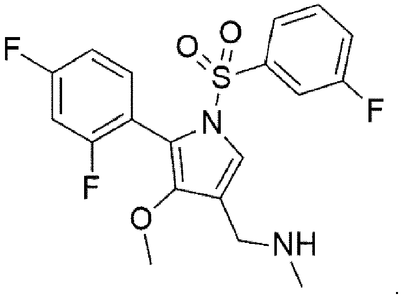

2. The liquid pharmaceutical composition according to claim 1, wherein:
the beta-cyclodextrin is (2-hydroxypropyl)-beta-cyclodextrin, or sulfobutyl ether-beta-cyclodextrin.

3. The liquid pharmaceutical composition according to claim 1, wherein:
the cyclodextrin is contained in an amount of 4.5 to 15.0 parts by weight based on 1 part by weight of the compound represented by Chemical Formula 1 or a pharmaceutically acceptable salt thereof.

4. The liquid pharmaceutical composition according to claim 1, wherein:
the bulking agent for freeze-drying is contained in an amount of 4.5 to 13.0 parts by weight based on 1 part by weight of the compound represented by Chemical Formula 1 or a pharmaceutically acceptable salt thereof.

5. The liquid pharmaceutical composition according to claim 1, wherein:
the bulking agent for freeze-drying is contained in an amount of 0.5 to 5.0 parts by weight based on 1 part by weight of the cyclodextrin.

6. The liquid pharmaceutical composition according to claim 1, wherein:
the bulking agent for freeze-drying is contained in an amount of 0.8 to 2.3 parts by weight based on 1 part by weight of the cyclodextrin.

7. The liquid pharmaceutical composition according to claim 1, wherein:
the liquid pharmaceutical composition is an injectable pharmaceutical composition.

8. The liquid pharmaceutical composition according to claim 1, wherein:
pH of the liquid pharmaceutical composition is 3.0 to 7.0.

9. The liquid pharmaceutical composition according to claim 1, wherein:
pH of the liquid pharmaceutical composition is 4.0 to 6.0.

10. The liquid pharmaceutical composition according to claim 1, wherein:
the compound represented by Chemical Formula 1 or a pharmaceutically acceptable salt thereof is contained at a concentration of 1 to 30 mg/mL in the liquid pharmaceutical composition.

11. The liquid pharmaceutical composition according to claim 1, wherein:
the compound represented by Chemical Formula 1 or a pharmaceutically acceptable salt thereof is contained at a concentration of 5 to 25 mg/mL in the liquid pharmaceutical composition.

## Patentansprüche

1. Flüssige pharmazeutische Zusammensetzung, die eine Verbindung, die durch die folgende chemische Formel 1 dargestellt wird, oder ein pharmazeutisch akzeptables Salz davon; ein Füllmittel für die Gefriertrocknung; Cyclodextrin; und ein Lösungsmittel umfasst,
wobei
das Cyclodextrin beta-Cyclodextrin oder gamma-Cyclodextrin ist,
das Cyclodextrin in einer Menge von 3,0 bis 25,0 Gewichtsteilen, auf Basis von 1 Gewichtsteil der Verbindung, die durch die chemische Formel 1 dargestellt wird, oder eines pharmazeutisch akzeptablen Salzes davon, enthalten ist,
das Füllmittel für die Gefriertrocknung D-Mannitol, Saccharose, Sorbitol oder Trehalose ist,
das Füllmittel für die Gefriertrocknung in einer Menge von 3,0 bis 25,0 Gewichtsteilen, auf Basis von 1 Gewichtsteil der Verbindung, die durch die chemische Formel 1 dargestellt wird, oder eines pharmazeutisch akzeptablen Salzes davon, enthalten ist,
das Lösungsmittel destilliertes Wasser, Wasser für Injektionszwecke, Acetatpuffer oder physiologische Kochsalzlösung ist, und
die [chemische Formel 1] ist

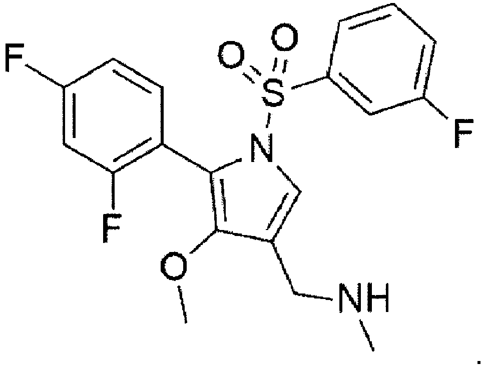

2. Flüssige pharmazeutische Zusammensetzung gemäß Anspruch 1, wobei:
das Beta-Cyclodextrin (2-Hydroxypropyl)-beta-Cyclodextrin oder Sulfobutylether-beta-Cyclodextrin ist.

3. Flüssige pharmazeutische Zusammensetzung gemäß Anspruch 1, wobei:
das Cyclodextrin in einer Menge von 4,5 bis 15,0 Gewichtsteilen, auf Basis von 1 Gewichtsteil der Verbindung, die durch die chemische Formel 1 dargestellt wird, oder eines pharmazeutisch akzeptablen Salzes davon, enthalten ist.

4. Flüssige pharmazeutische Zusammensetzung gemäß Anspruch 1, wobei:
das Füllmittel für die Gefriertrocknung in einer Menge von 4,5 bis 13,0 Gewichtsteilen, auf Basis von 1 Gewichtsteil der Verbindung, die durch die chemische Formel 1 dargestellt wird, oder eines pharmazeutisch akzeptablen Salzes davon, enthalten ist.

5. Flüssige pharmazeutische Zusammensetzung gemäß Anspruch 1, wobei:
das Füllmittel für die Gefriertrocknung in einer Menge von 0,5 bis 5,0 Gewichtsteilen, auf Basis von 1 Gewichtsteil des Cyclodextrins, enthalten ist.

6. Flüssige pharmazeutische Zusammensetzung gemäß Anspruch 1, wobei:
das Füllmittel für die Gefriertrocknung in einer Menge von 0,8 bis 2,3 Gewichtsteilen, auf Basis von 1 Gewichtsteil des Cyclodextrins, enthalten ist.

7. Flüssige pharmazeutische Zusammensetzung gemäß Anspruch 1, wobei:
die flüssige pharmazeutische Zusammensetzung eine injizierbare pharmazeutische Zusammensetzung ist.

8. Flüssige pharmazeutische Zusammensetzung gemäß Anspruch 1, wobei:
der pH-Wert der flüssigen pharmazeutischen Zusammensetzung 3,0 bis 7,0 beträgt.

9. Flüssige pharmazeutische Zusammensetzung gemäß Anspruch 1, wobei:
der pH-Wert der flüssigen pharmazeutischen Zusammensetzung **4,0** bis 6,0 beträgt.

10. Flüssige pharmazeutische Zusammensetzung gemäß Anspruch 1, wobei:
die Verbindung, die durch die chemische Formel 1 dargestellt wird, oder ein pharmazeutisch akzeptables Salz davon in einer Konzentration von 1 bis 30 mg/ml in der flüssigen pharmazeutischen Zusammensetzung enthalten ist.

11. Flüssige pharmazeutische Zusammensetzung gemäß Anspruch 1, wobei:
die Verbindung, die durch die chemische Formel 1 dargestellt wird, oder ein pharmazeutisch akzeptables Salz davon in einer Konzentration von 5 bis 25 mg/ml in der flüssigen pharmazeutischen Zusammensetzung enthalten ist.

## Revendications

1. Composition pharmaceutique liquide comprenant un composé représenté par la Formule Chimique 1 suivante, ou un sel pharmaceutiquement acceptable de celui-ci ; un agent de charge pour lyophilisation ; de la cyclodextrine ; et un solvant,
dans laquelle
la cyclodextrine est la bêta-cyclodextrine ou la gamma-cyclodextrine,
la cyclodextrine est présente en une quantité allant de 3,0 à 25,0 parties en poids par rapport à 1 partie en poids du composé représenté par la Formule Chimique 1 ou un sel pharmaceutiquement acceptable de celui-ci,
l'agent de charge pour lyophilisation est le D-mannitol, le saccharose, le sorbitol ou le tréhalose,
l'agent de charge pour lyophilisation est présent en une quantité allant de 3,0 à 25,0 parties en poids par rapport à 1 partie en poids du composé représenté par la Formule Chimique 1 ou un sel pharmaceutiquement acceptable de celui-ci,
le solvant est de l'eau distillée, de l'eau pour préparations injectables, un tampon acétate ou une solution saline physiologique, et
[Formule Chimique 1] est

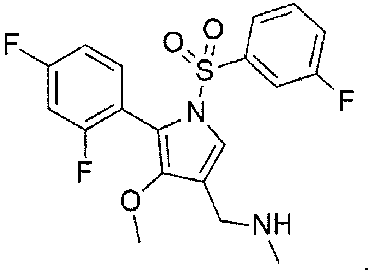

2. Composition pharmaceutique liquide selon la revendication 1, dans laquelle :
la bêta-cyclodextrine est la (2-hydroxypropyl)-bêta-cyclodextrine, ou l'éther sulfobutylique-bêta-cyclodextrine.

3. Composition pharmaceutique liquide selon la revendication 1, dans laquelle :
la cyclodextrine est présente en une quantité allant de 4,5 à 15,0 parties en poids par rapport à 1 partie en poids du composé représenté par la Formule Chimique 1 ou un sel pharmaceutiquement acceptable de celui-ci.

4. Composition pharmaceutique liquide selon la revendication 1, dans laquelle :
l'agent de charge pour lyophilisation est présent en une quantité allant de 4,5 à 13,0 parties en poids par rapport à 1 partie en poids du composé représenté par la Formule Chimique 1 ou un sel pharmaceutiquement acceptable de celui-ci.

5. Composition pharmaceutique liquide selon la revendication 1, dans laquelle :
l'agent de charge pour lyophilisation est présent en une quantité allant de 0,5 à 5,0 parties en poids par rapport à 1 partie en poids de la cyclodextrine.

6. Composition pharmaceutique liquide selon la revendication 1, dans laquelle :
l'agent de charge pour lyophilisation est présent en une quantité allant de 0,8 à 2,3 parties en poids par rapport à 1 partie en poids de la cyclodextrine.

7. Composition pharmaceutique liquide selon la revendication 1, dans laquelle :
la composition pharmaceutique liquide est une composition pharmaceutique injectable.

8. Composition pharmaceutique liquide selon la revendication 1, dans laquelle :
le pH de la composition pharmaceutique liquide est compris entre 3,0 et 7,0.

9. Composition pharmaceutique liquide selon la revendication 1, dans laquelle :
le pH de la composition pharmaceutique liquide est compris entre 4,0 et 6,0.

10. Composition pharmaceutique liquide selon la revendication 1, dans laquelle :
le composé représenté par la Formule Chimique 1 ou un sel pharmaceutiquement acceptable de celui-ci est présent à une concentration allant de 1 à 30 mg/mL dans la composition pharmaceutique liquide.

11. Composition pharmaceutique liquide selon la revendication 1, dans laquelle :
le composé représenté par la Formule Chimique 1 ou un sel pharmaceutiquement acceptable de celui-ci est présent à une concentration allant de 5 à 25 mg/mL dans la composition pharmaceutique liquide.
